Europäisches Patentamt

(19) European Patent Office

Office européen des brevets

(11) Numéro de publication : **0 218 488**
**B1**

(12) **FASCICULE DE BREVET EUROPÉEN**

(45) Date de publication du fascicule du brevet :
24.01.90

(51) Int. Cl.⁵ : **C 12 N 5/00**// C12N13/00

(21) Numéro de dépôt : **86401711.6**

(22) Date de dépôt : **31.07.86**

(54) **Procédé de culture des hépatocytes et co-cultures d'hépatocytes et de cellules épithéliales de foie traitées.**

(30) Priorité : 31.07.85 FR 8511714

(43) Date de publication de la demande :
15.04.87 Bulletin 87/16

(45) Mention de la délivrance du brevet :
24.01.90 Bulletin 90/04

(84) Etats contractants désignés :
AT BE CH DE FR GB IT LI LU NL SE

(56) Documents cités :
WO--A--81 /036 63
BIOLOGICAL ABSTRACTS, vol. 78, no. 7, 1984, page 5615, résumé no. 49889; A. GUILLOUZO et al.: "Long term production of acute-phase proteins by adult rat hepatocytes co-cultured with another liver cell type in serum-free medium", & BIOCHEM. BIOPHYS. RES. COMMUN. 120(2): 311-317
CHEMICAL ABSTRACTS, vol. 98, no. 15, 11 avril 1983, page 433, résumé no. 123537m, Columbus, Ohio, US; C. GUGUEN-GUILLOUZO et al.: "Maintenance and reversibility of active albumin secretion by adult rat hepatocytes cocultured with another liver epithelial cell type", & EXP CELL RES. 1983, 143(1), 47-54
BIOLOGICAL ABSTRACTS, vol. 70, no. 6, 1980, page 3723, résumé no. 35588; J.-C. WANSON et al.: "Interaction of adult rat hepatocytes and sinusoidal cells in coculture", & BIOL. CELL. 36(1); 7-16, 1979 [recd. 1980]

(73) Titulaire : IRE-CELLTARG S.A.
Zone Industrielle
B-6220 Fleurus (BE)

(72) Inventeur : Pirson, Philippe
180, Boulevard du Lambermont
B-1030 Bruxelles (BE)
Inventeur : Claikens, Vinciane
1, rue du Baty
B-5924 Thorembais Sainttrond (BE)
Inventeur : Schneider, Yves-Jacques
14 Benenboslaan
B-1900 Overyse (BE)

(74) Mandataire : Warcoin, Jacques et al
Cabinet Régimbeau 26, avenue Kléber
F-75116 Paris (FR)

**Description**

Les cultures primaires d'hépatocytes pris directement à partir de foies normaux et intacts, représentent une approche attirante pour l'étude des fonctions hépatocellulaires spécifiques chez l'animal ou chez l'homme.

Les hépatocytes du rat adultes, ou plus récemment de l'homme, peuvent être isolés facilement par des méthodes de perfusion du foie. Les cellules isolées doivent s'attacher dans le but de survivre pendant plus de quelques heures. Dans des conditions de culture conventionnelles, les cellules peuvent survivre pendant au maximum 1 à 2 semaines, mais elles perdent rapidement leurs fonctions hautement différenciées (Ref. 1) par exemple :

(a) la sécrétion de protéines sériques, incluant l'albumine, la transferrine, la glycoprotéine acide $\alpha_1$ et la globuline ;

(b) les biotransformations complètes de médicaments et autres xénobiotiques en métabolites hydrosolubles par formation de sels biliaires ;

(c) les formes iso-enzymes adultes ;

(d) la réponse enzymatique à des hormones spécifiques ;

(e) les récepteurs membranaires spécifiques pour la reconnaissance des protéines extracellulaires telles que les asialoglycoprotéines.

Par conséquent, la régulation des fonctions hépatiques, en particulier les activités enzymatiques, ne peut pas être étudiée pendant plus de quelques jours dans un système conventionnel de culture.

Dans le but d'améliorer le maintien en culture du phénotype des hépatocytes normaux, différentes approches ont été considérées. L'alimentation sélective du milieu en suppléments tels que les hormones (insuline, glucagon, glycocorticoïdes...) et tels que des facteurs de croissance, ont certains effets sur la viabilité et les fonctions des hépatocytes. Les composés de la matrice extracellulaire ont aussi été examinés. La fixation cellulaire et la survivance ont été améliorées en étalant les cellules sur différents collagènes (Ref. 2), de la fibronectine (Ref. 3), sur une biomatrice connective du foie (Ref. 4), sur un matériau extracellulaire secrété par les cellules endothéliales de cornée de bœuf (Ref. 5) ainsi que sur des fibroblastes (Ref. 6), des cellules embryonnaires (Ref. 7), ou des cellules endothéliales sinusoïdales homologues (Ref. 8). Cependant, indépendamment des conditions de culture, les changements phénotypiques des hépatocytes ont conduit à une perte partielle ou complète des fonctions hautement différenciées après plusieurs jours de culture.

La présente invention concerne un procédé de culture des hépatocytes, permettant de maintenir l'intégralité de leurs fonctions spécifiques, caractérisé en ce que lesdits hépatocytes sont mis en co-culture avec des cellules de foie de type épithélial dont les capacités de division ont été définitivement inhibées par un traitement approprié.

Il peut s'agir d'un traitement physique, tel que l'irradiation ou la chaleur : en particulier, les radiations gamma peuvent être utilisées.

Il peut s'agir d'un traitement chimique : des drogues antimitotiques peuvent être utilisées à ce titre, et en particulier, la mitomycine C.

En effet, des observations récentes (Ref. 8-12) ont démontré que les hépatocytes de rat ou d'homme mis en culture conjointement avec une lignée cellulaire épithéliale de foie de rat, probablement dérivée de cellules des canalicules biliaires, maintiennent, pendant plusieurs jours et à un niveau important, les fonctions hépatiques spécifiques, telles que la sécrétion de l'albumine ou la métabolisation de médicaments par des voies variées typiques du processus de biotransformation.

Une caractéristique importante du système de co-culture est sa grande spécificité, puisque les cellules épithéliales doivent avoir une origine hépatique (probablement issues du canal de Hering) et ne pourraient pas être remplacées par un milieu conditionné ou par un extrait cellulaire (13). Ce système n'est pas spécifique de l'espèce, puisque des résultats comparables sont obtenus lorsque des hépatocytes humains sont mis en culture conjointement avec les cellules épithéliales de foie de rat (Ref. 10), et ce système est corticostéroïde-dépendant (Ref. 14, 15).

Néanmoins, ce système présente un problème majeur qui est la lignée cellulaire épithéliale elle-même. Cette lignée cellulaire dérive d'un foie de rat nouveau-né, et est obtenue après une digestion du foie par la trypsine. Sous certaines conditions, certaines cellules se divisent, forment des colonies et peuvent être repiquées. Les cellules se développent lentement en culture. Cependant, après plusieurs passages, elles subissent une transformation progressive et présentent des caractéristiques de cellules cancéreuses. Les cellules épithéliales transformées de rat perdent leurs propriétés et avantages de co-culture sur les hépatocytes (Ref. 13).

C'est pourquoi, la présente invention concerne une méthodologie originale qui permet tous les avantages du système de co-culture mais utilise des cellules épithéliales transformées (à des degrés variés) disponibles commercialement à l'A.T.C.C. ou d'autres collections. De toutes les cellules de foie de type épithélial, les cellules de foie Chang ont été sélectionnées pour leurs caractéristiques de croissance : division rapide, croissance en monocouche, fixation cellulaire, etc... Pour prévenir leur multiplication, néfaste aux avantages de co-culture, les cellules de foie Chang ont été soumises à des radiations gamma et/ou à des drogues antimitotiques. Ce traitement spécial a permis de maintenir les cellules dans un état de non croissance et de les cultiver conjointement avec des hépatocytes de rat.

De la même façon, les cellules de foie Chang peuvent être cultivées conjointement avec des hépatocytes humains.

Le procédé de co-culture selon la présente invention, comprend les étapes suivantes dans lesquelles les hépatocytes peuvent être d'origine murine ou humaine :

(a) Préparation des milieux de cultures :
milieu de culture des cellules de type épithélial, ou milieu épithélial,
milieu de culture des hépatocytes.
(b) Culture des cellules de foie Chang.
(c) Préparation des cellules de foie Chang pour les expériences de co-culture.
(d) Isolement des hépatocytes.
(e) Mono-culture des hépatocytes (étape à visée comparative).
(f) Co-culture des hépatocytes avec les cellules de Chang.

Les abréviations suivantes seront utilisées au cours de la description :

HEPES = Acide 4-2-hydroxyéthyl-1-pipérazine-éthane sulfonique.
EGTA = Acide éthylène-glycol-bis-(β-aminoéthyléter)-tétra acétique.
FCS = Sérum de fœtus de veau (inactivé par la chaleur à 56 °C pendant 30 minutes).
HBSS = Solution saline équilibrée de Hanks (Hanks balanced saline solution).

Les origines des différents matériaux utilisés sont répertoriées dans le tableau suivant :

| MATERIAUX | ORIGINE |
|---|---|
| Poudre du Milieu, Sérum, Solution Trypine-EDTA, antibiotiques. | Laboratoires GIBCO (Grand Island, N.Y.) |
| Solution de collagène (vitrogen 100). | Laboratoires FLOW |
| Produits chimiques, insuline de boeuf, albumine de rat, mitomycine-C. | Sigma Chemical Co. (St Louis, Mo) |
| Hemisuccinate d'hydrocortisone | Roussel (Paris, France) |
| Collagénase type IV | Boehringer (Mannheim, FRG) |
| Flacons de culture tissulaire | Falcon (Becton Dickinson, Oxnard, Ca) |
| Boîtes de Petri en plastique (20cm$^2$) (Lux Contur) | Labo Miles (Naperville, I1.) |
| Boîtes Petriperm perméables aux gaz (20cm$^2$) | Heraeus (Hanau, FRG) |
| Collagénase type CLS II | Worthington (Copper Company) |

(a) Les milieux de cultures

Le milieu épithélial a été constitué à partir d'un milieu minimum essentiel (sel Earl) contenant 25 mM de NaHCO$_3$, 20 mM de Hepes, 2 mM de glutamine, 100 µg/ml de streptomycine, 100 U/ml de pénicilline et additionné de 1 % d'une solution d'acides aminés non essentiels et de 10 % de sérum de fœtus de veau inactivé par la chaleur (FCS).

Le milieu pour la culture des hépatocytes de rat ou humains (DE milieu) a consisté en un milieu de Dulbecco modifié par Eagle contenant 40 mM de NaHCO$_3$, 20 mM de HEPES, 4 mM de glutamine, 20 mM de glucose, 100 µg/ml de streptomycine, 100 U/ml de pénicilline. Ce milieu a été additionné de 10 % de FCS, 10 µg/ml d'insuline et 5 x 10$^{-5}$ M d'hémisuccinate d'hydrocortisone (milieu de mono-culture) ou 3,5x10$^{-6}$ M d'hémisuccinate d'hydrocortisone (milieu de co-culture).

(b) La culture des cellules de foie Chang :

La lignée cellulaire hépatique Chang a été établie à partir de foie humain non malin en 1954 par R.S. Chang (Proc. Soc. Exp. Biol. Med., 87, 440, 1954) et a été soumise à l'A.T.C.C. sous le N° ATCC CCL13. La lignée consiste en cellules de foie de type épithélial croissant en monocouche.

Les cellules ont été cultivées à 37 °C dans une atmosphère saturée en eau sous le rapport CO$_2$/air (10 : 90, v/v) dans des flacons de culture tissulaire en plastique de 175 cm$^2$ (T175), et le milieu épithélial a été renouvelé 3 fois par semaine. Les cultures ont été repiquées dès confluence cellulaire tous les 7-9 jours. Les cultures repiquées ont été préparées par trypsinisation des monocouches de cellules, avec un mélange trypsine-EDTA comprenant 0,05 % (poids/volume) de trypsine et 0,02 % (poids/volume) d'EDTA. Pour ce faire, le milieu de la culture a été enlevé, les cellules lavées avec une solution saline contenant le tampon de phosphate 10 mM, pH 7,4 et incubées 10 minutes à 37° avec la solution trypsine-EDTA. Les cellules ont été remises en suspension dans le milieu épithélial, et les agrégats cellulaires ont été décomposés par prélèvement vigoureux et répété à la pipette de la suspension cellulaire. La suspension cellulaire a été déposée dans de nouveaux flacons avec un rapport de repiquage de 1 : 10.

(c) Préparation des cellules de foie Chang pour les expériences de co-culture

Préalablement à la co-culture avec les hépatocytes de rat, les cellules de foie Chang ont été soumises à l'irradiation, aux antimitotiques (exemple mitomycine C) ou aux deux, dans le but d'inhiber leur prolifération et de les conserver en monocouche.

Les cellules confluentes ont été remises en suspension par traitement de trypsine-EDTA ainsi que décrit pour le repiquage. Les cellules ont été diluées avec du milieu épithélial, centrifugées 5 minutes à 400 × g et remises en suspension dans le milieu de co-culture. Après comptage, le nombre de cellules a été ajusté à 5 x 10$^6$ cellules/ml avec le même milieu.

Les tubes contenant 2 ml de cette suspension cellulaire ont été irradiés par une source de Césium 237 à une dose variant entre 1 000 à 4 000 rads.

Pour le traitement cellulaire avec les antimitotiques, 2 ml de la suspension cellulaire (10$^7$ cellules au total) ont été transférés dans des tubes de 15 ml et mélangés avec la mitomycine-C (Sigma) et le milieu de co-culture, pour obtenir une concentration de 5 à 15 µg de médicament/ml dans un volume total de 5 ml par tube. La concentration finale de cellules a été ajustée à 2 x 10$^6$ par ml. Le mélange a été incubé 30 minutes à 37 °C avec des agitations fréquentes.

Dans le cas d'un traitement mixte, les cellules ont été successivement irradiées à une dose de 500 ou 1 000 rads et incubées avec 5 µg de mitomicine-C/ml ainsi que décrit ci-dessus.

Après chaque traitement, les cellules ont été lavées une fois dans le milieu et cultivées pendant 24 heures dans des flacons T 175 (un flacon par tube) avec le milieu de co-culture.

Une heure avant la co-culture avec les hépatocytes de rat, les cellules ont été remises en suspension dans le milieu de co-culture après trypsination et comptées dans un hémocytomètre. Le nombre de cellules viables a été déterminé par le test d'exclusion du bleu trypan. Les cellules ont été diluées dans le même milieu pour obtenir 0,6 x 10$^6$ cellules vivantes/ml.

Les cellules de foie Chang pour les expériences de co-culture avec les hépatocytes humains ont seulement été soumises à l'irradiation. Dans le cas des hépatocytes humains, les cellules de foie Chang confluentes sont irradiées à une dose de 3 000 rads et ce directement dans leur boîte de culture. Les cellules sont ensuite remises en suspension, comptées, puis co-cultivées comme on l'a décrit pour les hépatocytes de rat.

(d) Isolement des hépatocytes

Isolement des hépatocytes de rat :

Les hépatocytes de rat ont été isolés à partir de rats Wistar de 250 g par perfusion du foie avec une solution de collagénase, ainsi que décrit par Limet et al. (Eur. J. Biochem, 1982, 125, 437-443).

Brièvement, le foie a été d'abord perfusé avec 300 ml d'une solution tampon née de Hanks ne contenant pas d'ions Ca$^{2+}$, additionnée de 0,5 mM EGTA, à un débit de 30 ml/min et oxygénée de façon continue avec un mélange de O$_2$/CO$_2$ (95 : 5 ; v/v). Le foie a alors été perfusé avec 300 ml de la solution de Hanks additionnée de 2,5 mM de calcium, 180 mg de collagénase type IV (Boehringer, Mannheim, FRG) sous oxygénation. Après 30 minutes, les cellules ont été davantage dissociées pendant 15 minutes dans 100 ml de 0,1 % (p/v) de collagénase dans la solution de Hanks à 37 °C sous oxygénation. La suspension cellulaire a été filtrée à travers des filtres de perlon successifs de taille de pore 100 µm, 63 µm et 28 µm, puis a été laissée sédimenter pendant 20 minutes sur de la glace. Après avoir éliminé le surnageant contenant les débris cellulaires ainsi que les cellules sanguines et sinusoïdales, le culot a été lavé 3 fois par centrifugation 2 minutes à 50 x g avec le milieu de culture complet (milieu DE additionné de 10 % FCS) refroidi dans de la glace.

La viabilité des cellules a été estimée par le test d'exclusion du bleu trypan sous microscopie par contraste de phase. Après comptage, les hépatocytes ont été mis en suspension à 10$^6$ cellules/ml dans le milieu de culture complet.

Isolement des hépatocytes humains :

Les échantillons de foie humain proviennent soit de donneurs rénaux dont le foie ne peut être utilisé pour une transplantation (cause endogène, absence de receveur etc...), soit de patient ayant subi une hépatectomie partielle (résection au cours d'une greffe de foie, d'un carcinome primaire ou secondaire, etc.). Le matériel de transplantation est préalablement perfusé avec une solution saline froide, riche en potassium (type Euro-Collins). Le matériel de résection est plongé dans la même solution après intervention chirurgicale. Les échantillons sont conservés dans de la glace jusqu'au moment de leur utilisation.

L'extrémité des lobes hépatiques, le petit lobe caudal ou toute portion de foie dont au moins deux faces sont ouvertes par la capsule de Glisson sont utilisés pour l'isolement des hépatocytes. Les cellules sont dissociées par perfusion à la collagénase d'une ou plusieurs branches de la veine portale identifiées sur la section du lobe et cannulées.

Brièvement, les morceaux de foie sont successivement perfusés sans recirculation 10 min avec la solution saline de Hanks (pH 7,2), sans calcium ni magnésium et contenant 25 mM d'HEPES (HBSS), additionnée de 0,5 mM EGTA, puis 10 min avec le milieu HBSS sans EGTA à un débit de 100 ml/min et oxygénés de façon continue avec un mélange de $O_2/CO_2$ (95 : 5 ; v/v). Pour compenser la déperdition de chaleur due au système de perfusion, l'eau de circulation est maintenue à 38-40°. Les morceaux de foie sont ensuite perfusés avec le milieu HBSS contenant 5 mM de $CaCl_2$ et 0,05 % de collagénase (Worthington, type CLS II, 140-160 μ/mg) (solution de dissociation). La solution de perfusion est recirculée quelques minutes après le début de la dissociation. Le volume total utilisé est de 500 ml. La dissociation est poursuivie pendant 25 à 45 min. Les morceaux de foie sont ensuite transférés dans une boîte de Pétri contenant la solution de dissociation. Les morceaux sont débarrassés de leur capsule, et les cellules dissociées sont dispersées au moyen d'une spatule ou d'une pince. recourbée. La suspension cellulaire ainsi obtenue est transférée dans une bouteille de 100 ml, gazéifiée durant 3 min puis filtrée sur un filtre de 100 μm. Les agrégats et morceaux non dissociés sont éventuellement réincubés 15 min à 37° dans 100 ml de solution de dissociation, puis refiltrés. La suspension cellulaire est ensuite passée successivement sur un filtre de 60 μm et 28 μm. Les cellules sont ensuite centrifugées 3 min à 50 g puis lavées deux fois avec du milieu DE contenant 10 % de FCS (1 min 45 sec x 50 g).

Par cette méthode on obtient des hépatocytes viables à plus de 90 % avec un rendement cellulaire de 5 à 20 x $10^6$ hépatocytes par gramme de foie.

(e) Les hépatocytes en mono-culture

Cas des hépatocytes de rat :

Pour les cultures conventionnelles (monocultures), les hépatocytes de rat ont été étalés dans des boîtes de Petriperm de 20 cm² ou des boîtes de Petri en plastique, recouvertes de collagène, à une densité de 3-3, 3 x $10^6$ cellules par boîte. Cette couverture a été obtenue en incubant les boîtes de Petriperm pendant 16 heures à 37° avec 2,5 ml de collagène de peau de veau soluble (Vitrogen 100) à 15 μg/ml dans une solution saline tamponnée de phosphate 10 mM pH 7,4 et en les lavant postérieurement avec le même tampon. Pour les boîtes en plastique, 0,5 ml de solution de collagène (60 μg/ml dans une solution saline de HCl 0,013 M) ont été versés dans chaque boîte, laissés séchés pendant la nuit à 37 °C et rincés avec du milieu de culture une heure avant l'utilisation. Les cellules ont été cultivées à 37 °C dans une atmosphère saturée en eau sous le rapport $CO_2$/air (10 : 90 ; p/v). Après 3-4 heures, le temps nécessaire pour la fixation cellulaire, le milieu a été écarté (avec les cellules non attachées ou mortes) et remplacé par 2,5 ml de milieu mono-culture par boîte. Pendant les heures suivantes, les hépatocytes ont continué de s'agréger pour former, après 20 heures, une monocouche avec des travées de type hépatique et des canalicules biliaires ainsi que l'a montré l'examen sous microscopie à contraste de phase et lumière. Le milieu de culture a été renouvelé quotidiennement et conservé congelé pour les analyses suivantes.

Cas des hépatocytes humains :

Les hépatocytes humains isolés par la méthode décrite ci-dessus sont cultivés selon le système développé pour les hépatocytes de rat.

Les hépatocytes humains ont été étalés sur 20 cm² dans des boîtes de Petri en plastique, recouvertes de 100 μg de collagène (vitrogen 100) et de 50 μg de fibronectine humaine par boîte. La densité cellulaire est de 5 x $10^6$ cellules par boîte dans le système conventionnel (mono-culture).

(f) Les hépatocytes en co-culture avec les cellules de foie Chang

Pour la co-culture, 1,8 à 2 x $10^6$ cellules parenchymateuses isolées ont été ensemencées dans les mêmes conditions que les cultures conventionnelles. Après 4 heures, le milieu a été remplacé par 1,5 x $10^6$ cellules épithéliales de foie Chang (irradiées et/ou traitées par la mitomycine C) suspendues dans 2,5 ml du milieu de co-culture. Les hépatocytes ont continué de s'agréger, de s'étendre et de former des cordons de cellules pendant que les cellules épithéliales ont occupé toutes les surfaces libres. Les hépatocytes de rat ont été cultivés dans les mêmes conditions que pour les monocultures. Dans le cas des hépatocytes humains, la densité cellulaire est de 3 x $10^6$ cellules par boîte pour le système de co-culture.

En conclusion, dans les conditions de culture conventionnelle, les hépatocytes ne survivent pas pendant plus de deux semaines et perdent leurs fonctions hautement différenciées. Les associations en co-culture des hépatocytes avec un autre type cellulaire de foie probablement dérivé des cellules de canalicules biliaires, maintiennent à des forts pourcentages les fonctions différenciées des hépatocytes. L'association des hépatocytes avec une lignée cellulaire de type épithélial de foie humain, transformée lors de nombreux passages, mais soumise à un traitement spécial, donne une amélioration identique du maintien des activités spécifiques des hépatocytes. Ce système de co-culture original permettra des études scientifiques particulières telles que la physiologie hépatique, la toxicité des drogues et la carcinogenèse hépatique ou permettra la préparation d'un produit particulier tel qu'un système de culture in vitro pour des micro-organismes (parasites protozoaires ou virus).

Ce système de co-culture pourrait aussi servir de moyen de diagnostic.

Enfin, ces co-cultures peuvent être conservées dans des conditions connues de l'homme du métier, telles que le froid, et sont ainsi, par conséquent, directement utilisables.

Les avantages et particularités du procédé selon la présente invention sont étudiés plus en détail dans les exemples suivants.

I — Cas des hépatocytes de rat

Exemple 1

Maintien des caractéristiques morphologiques et de la survivance cellulaire

Toutes les perfusions de foie de rat ont produit des cultures réussies et ont donné des résultats similaires dans les cultures conventionnelles et mélangées. Une viabilité de 85 à 95 % a été obtenue usuellement par la technique de la dissociation à la collagénase in situ. Toutes les observations morphologiques ont été faites sur des préparations vivantes sous microscopie à contraste de phase ou sur des cellules colorées à l'hématoxyline-éosine par microscopie lumineuse.

En moins de trois heures, les hépatocytes isolés ont été attachés au substrat, ont été agrégés, et ont formé des monocouches de cellules. Les cellules ont adhéré, se sont disséminées et ont été cultivées de façon aussi efficace mais pendant plus longtemps sur des membranes en plastique que sur des membranes perméables au gaz (boîtes Petriperm) à partir desquelles le détachement cellulaire peut arriver après trois semaines de culture. Les concentrations d'hémisuccinate d'hydrocortisone, ajouté de façon routinière aux cultures conventionnelles et aux co-cultures, ont donné une survivance cellulaire maximum et une synthèse d'albumine maximum. Dans les expériences de co-culture, la concentration cellulaire la plus satisfaisante a été de $1,8 \times 10^6$ hépatocytes et de $1,5 \times 10^6$ cellules de foie Chang pour 20 cm$^2$ de boîte de Petri.

Dans les conditions de culture conventionnelle, la survie des hépatocytes n'a pas excédé 12 jours avec un milieu de culture complet et 20 jours avec un milieu de mono-culture (milieu contenant des hormones), bien que dans certaines préparations, certains cordons hépatocytaires développés autour de cellules contaminantes peuvent survivre plus longtemps. Les hépatocytes ont gardé leur structure typique, par exemple des cellules bien individualisées et polyédriques, avec un ou deux petits noyaux réfringents, pendant seulement 5 à 6 jours. Plus tard, ils ont dégénérés progressivement, pour ne former que des cellules de taille plus petite avec un large noyau et de nombreuses cellules multinucléées, puis ont disparu complètement après deux à trois semaines.

Quand les hépatocytes ont été cultivés en présence des cellules de foie Chang traitées (par les trois procédures), après ensemencement, une monocouche cellulaire confluente a été observée en moins de 24 heures. Les cellules épithéliales ont occupé les espaces libres entre les trabécules des hépatocytes et ont établi des contacts étroits avec ces dernières. Pendant plusieurs semaines (au moins quatre), les hépatocytes ont été organisés en trabécules ou en petits îlots, et ont maintenu leur morphologie typique cuboïdale polyédrique contenant 1 ou 2 petits noyaux ronds et réfringents incluant un nucléole dense, et un cytoplasme granulaire. Les canalicules biliaires ont été présents dans toutes les trabécules entre les hépatocytes. Les gouttelettes lipidiques ont été aussi trouvées et leur nombre a augmenté avec l'âge. Ce phénomène a été réversible lorsque le milieu a été renouvelé deux fois pendant une semaine au lieu de quotidiennement. Les hépatocytes en co-culture ont été généralement bien visibles mais des cellules mortes, des précipités de protéines sériques et des cellules de foie Chang détachées peuvent apparaître en superficie après une semaine de culture. Cet inconvénient peut être surmonté si des précautions spéciales ont été prises pendant l'isolement des hépatocytes (décantation après filtration cellulaire pour enlever les fibroblastes et autres cellules contaminantes) et les cultures cellulaires (filtration de FCS, agitation de chaque boîte ou lavage soigneux préalablement à l'alimentation pour enlever les débris cellulaires, alimentation du milieu à 37 °C et réduction de la fréquence d'alimentation à deux fois par semaine).

Les meilleurs essais comparatifs des cellules de foie Chang, sur les hépatocytes de rat en culture, ont été obtenus lorsque les cellules épithéliales humaines ont été soumises aux conditions suivantes :

1° irradiation à une dose de 3 000 rads (I)

2° incubation avec la mitomycine C à une concentration finale de 10 μg de médicaments/ml de milieu de co-culture (II),

3° irradiation à une dose de 500 rads suivie par une incubation avec 5 μg de mitomycine C/ml (III).

Ces conditions ont garanti le maintien des hépatocytes sains pendant au moins 4 semaines. Pour une période de culture plus longue, il est parfois nécessaire, après trois à quatre semaines de culture, d'additionner des cellules de foie Chang traitées fraîchement (pour les conditions I et II) ainsi que décrit préalablement mais à une densité plus basse ($10^6$ cellules/boîte), pour recouvrir l'espace, entre les hépatocytes, laissé libre par les cellules épithéliales mortes.

L'aspect morphologique des cellules de foie Chang, irradiées (condition I) ou soumises à un traitement mixte (condition III) a changé de façon drastique pendant la co-culture. Les cellules sont devenues larges avec deux noyaux ou plus pendant que le nombre de cellules a diminué avec le temps de culture. Moins de 30 % des cellules initialement ajoutées aux hépatocytes sont restées présentes dans la culture après trois semaines. Lorsque les cellules de foie Chang ont été traitées par la mitomycine C, les cellules ont gardé un aspect plus similaire aux cellules non traitées et environ 20 % des cellules sont restées viables trois semaines après l'étalement.

Si les cellules de foie normales Chang ont été ajoutées aux hépatocytes, les cellules ont grandi de façon continue pour recouvrir finalement toutes les trabécules hépatocytaires après 7 jours. Des effets similaires ont été fréquemment observés lorsque les cellules épithéliales ont été irradiées à des doses égales ou inférieures à 2 500 rads ou à des concentrations en mitomycine-C inférieures à 8 µg/ml.

Exemple 2

Maintien de la sécrétion d'albumine et de transferrine actives

1) Essais

La quantité d'albumine et de transferrine, sécrétées par les hépatocytes dans le milieu a été déterminée par immunonéphélométrie en utilisant un système auto-analyseur ABBOTT (Wiesbaden-Delkenheim, FRG). Les solutions standards d'albumine et de transferrine de rat et des milieux de culture ont été mélangés avec des dilutions appropriées d'anticorps de lapin anti-albumine de rat et anti-transferrine de rat dans un tampon phosphate de 0,1 M̂ contenant 4 % de polyéthylène glycole-6000 et ont été incubés à 25 °C pendant 12 minutes pour l'albumine et 30 minutes pour la transferrine avant une mesure par diffusion lumineuse. Les anticorps spécifiques étaient de la Corporation USB (Cleveland, Ohio). La limite inférieure de sensibilité de l'essai a été de 2 à 3 µg d'albumine ou de transferrine par ml.

2) Résultats

Lorsque les hépatocytes de rat adulte ont été cultivés dans les conditions conventionnelles avec un milieu complet, la sécrétion maximale d'albumine a été atteinte le jour suivant l'étalement. Par la suite, la moyenne de sécrétion a diminué rapidement pour atteindre au jour 4 une moyenne plus basse jusqu'à ce que les hépatocytes aient disparu de la culture (environ 10 jours). Un profil similaire dans la sécrétion de la transferrine a été observé avec la différence que la moyenne y restait extrêmement basse. Les taux de sécrétion d'albumine et de transferrine ont été similaires indépendamment du fait que les hépatocytes ont été étalés sur boîte de Petri en plastique ou sur boîte de Petriperm recouvertes de collagène.

Quand les hépatocytes ont été cultivés avec un milieu de mono-culture, les sécrétions en albumine et transferrine ont été maintenues à un haut niveau pendant plus de 10 jours, puis la sécrétion a décliné progressivement et est restée stable jusqu'à la mort des hépatocytes environ au 18ème jour.

La sécrétion d'albumine et de transferrine a été parfois observée après deux semaines. Dans ces préparations, peu d'hépatocytes sains ont pu être observés, encadrés par des cellules de type épithélial poussant abondamment en clones. Ces sécrétions de protéines peuvent être produites seulement par les hépatocytes ou en association avec les autres cellules contaminantes qui se développent et sont connues pour être capables d'exprimer certaines fonctions hépatiques telles que les productions de protéines plasmatiques (ref. 16).

En co-culture, la sécrétion optimale d'albumine a été obtenue après plusieurs jours de culture et atteint des niveaux plus élevés que ceux observés dans les cultures conventionnelles. Les moyennes de l'ordre de 25 µg à 75 µg de protéines sécrétées par $10^6$ hépatocytes en 24 heures de culture (10 µg à 25 µg de protéines/$10^6$ hépatocytes en 24 heures dans des cultures conventionnelles). Des vagues périodiques de sécrétion croissante de protéines ont été observées. Le taux de sécrétion tendait à décliner progressivement après 6 semaines de culture. Un profil similaire dans la sécrétion de la transferrine a été observé. Aucun changement significatif n'a été observé quelles que soient les conditions de préparation des cellules de foie Chang. On a trouvé que ni l'albumine ni la transferrine n'ont été sécrétées dans le milieu de culture pure de cellules de foie Chang (traitées ou pas) pendant deux semaines de culture.

Exemple 3

Sécrétion des enzymes solubles et de l'urée en tant qu'indicateurs de la viabilité des hépatocytes

1) Essai

Les enzymes solubles du cytosol tels que la lactate déhydrogénase (LDH), la gamma glutamyle transpeptidase (gamma-GT), la glutamyle oxaloacétate transaminase (GOT) et la glutamyle pyruvate transaminase (GPT), et l'urée présentes dans le milieu ont été déterminés en utilisant les kits de diagnostic spécifiques ABBOTT.

2) Résultats

Le test utilisé pour la sécrétion des enzymes solubles présents dans le cytoplasme et en conséquence libérés à la mort des cellules, n'a pas été spécialement fiable dans les expériences de co-culture.

La production d'urée a été un paramètre plus sensible pour évaluer la viabilité cellulaire. La production d'urée et sa sécrétion dans les milieux de culture reflètent directement l'état des cellules. Cette excrétion a diminué parallèlement à la sécrétion d'albumine et à l'observation morphologique dans les cultures conventionnelles. En contraste, l'urée a été excrétée à un niveau plus élevé et pendant de longues périodes dans le système de co-culture.

Exemple 4

Métabolisme médicamenteux

1) Essai

Les activités de biotransformation des médicaments exercées par les hépatocytes de rat dans les conditions conventionnelles ou de co-culture ont été déterminées en utilisant l'astémizole, un antihistaminique $H_1$ (Hiomanol de Janssen Pharmaceutica, Beerse, Belgique), en tant que médicament de référence. La métabolisation de ce médicament a été choisie pour l'étude du métabolisme médicamenteux de ces hépatocytes en culture, du fait que ses différents métabolites chez le rat sont bien connus.

Pratiquement, après différentes périodes de culture, les cellules ont été incubées pendant 24 heures à 37 °C en présence d'un milieu de culture de 2,5 ml contenant 10 µg/ml d'astémizole marqué au tritium. A la fin de la période d'incubation, le milieu de culture a été centrifugé 10 minutes à 1 000 x g pour faire partir les dépôts cellulaires et le surnageant restant a été traité pour une analyse HPLC. L'astémizole et ses métabolites présents dans les milieux de culture ont été déterminés par la technique HPLC utilisant un détecteur de radioactivité.

2) Résultats

La culture des hépatocytes de rat incubés avec l'astémizole (AST) 24 heures après l'ensemencement des cellules a procuré les métabolites suivants dans les milieux de culture : desméthyl-AST, 5-hydroxy-AST, 6-hydroxy-AST, 6-hydroxy-désméthyl-AST, leurs glucuronoconjugués, et des métabolites mineurs tels que le 4-hydroxy-α-4-méthoxy-phénylacétique ou phénylacéturique.

Ces métabolites différents correspondent à l'occurrence de voies métaboliques trouvées in vivo. Les voies de réduction, déméthylation et glucuronidation, qui sont les voies majeures, ont été utilisées pour estimer l'activité métabolique des hépatocytes dans la culture pure ou mélangée avec les cellules épithéliales.

Après 14 jours de culture conventionnelle, 70 % et 84 % de la drogue ont été métabolisés et la glucuronidation intervient pour seulement 20 % et 50 % de la drogue totale ajoutée lorsque les hépatocytes sont cultivés respectivement avec le milieu complet ou le milieu en mono-culture.

Lorsque les hépatocytes ont été co-cultivés par les cellules de foie Chang irradiées à une dose de 3 000 rads, plus de 90 % de l'astémizole a encore été conjuguée en tant que glucuronide après deux semaines de culture.

Dans la culture pure des cellules de foie Chang non traitées, moins de 23 % de la drogue a été métabolisée après 24 heures d'incubation mais les conjugués glucuronés n'ont jamais été formés.

Exemple 5

Maintien de l'activité des récepteurs membranaires spécifiques des asialoglycoprotéines

1) Essai

Après différentes périodes de culture, les monocouches cellulaires ont été incubées avec 1 ml de milieu de mono-ou de co-culture contenant 20 µg/ml d'albumine sérique humaine marquée au tritium ou d'albumine humaine galactosylée. Le marquage et la galactosylation de l'albumine humaine ont été faits comme décrit préalablement (Schneider et al, 1984. dans : Ciblage des médicaments par l'intermédiaire des récepteurs, Grégoriadis G.,Poste G. Senior J. and Trouet A., eds, Plenum Press, pp. 1-25).

Après 24 heures d'incubation à 37 °C, les milieux de culture ont été testés pour la présence de produits de dégradation après précipitation des protéines par 25 % (p/v) d'acide trichloroacétique (concentration finale) pendant 30 minutes à 4 °C, centrifugation 30 minutes à 1 000 x g et essai du surnageant pour la radioactivité.

Parallèlement, les cellules ont été lavées à 4 °C, deux fois avec 2,5 ml d'une solution saline tamponnée par du phosphate 10 mM pH 7,4, une fois avec 2,5 ml du milieu de culture complet, trois fois avec 2,5 ml de la même solution saline tamponnée par du phosphate, dissoutes dans 1 ml de 1 % (p/v) de déoxycholate de sodium à un pH de 11,3 et testées pour, et la radioactivité, et la concentration en protéines cellulaires.

La capture spécifique représente la somme de l'accumulation (protéines associées aux cellules) et de la digestion (produits de dégradations marqués présents dans le milieu).

2) Résultats

Dans les organismes vivants, les glycoprotéines désyalylées ou les protéines galactosylées (néoglyco-protéines) sont sélectivement reconnues par les hépatocytes et captées par endocytose par l'intermédiaire des récepteurs cellulaires (17). Ces récepteurs sont exprimés de façon spécifique par les hépatocytes. Le maintien de ces fonctions hautement spécifiques des hépatocytes a été testé dans des systèmes conventionnels et de co-culture utilisant l'albumine humaine native ou galactosylée marquée au tritium. Les résultats de trois expériences ont été exprimés sous forme du rapport de capture de l'albumine galactosylé sur le captage de l'albumine (rapport spécifique).

Après 24 heures d'incubation en présence d'hépatocytes vieux d'un jour, le rapport spécifique a été de 120 ou de 250 lorsque les hépatocytes ont été alimentés respectivement avec un milieu contenant ou pas des hormones. En culture conventionnelle avec les milieux complets et de monoculture, le rapport spécifique tombe rapidement à des valeurs inférieures à 4 après huit jours de culture. A huit jours de culture, le rapport spécifique a été supérieur à 50 dans le système de co-culture avec les cellules de foie Chang irradiées à 3 000 rads et est resté élevé pendant au moins 2 semaines.

Les cellules de foie Chang toutes seules (irradiées ou non) n'ont pas les récepteurs spécifiques. Dans certaines préparations d'hépatocytes, le rapport spécifique a été plus bas que celui décrit mais toujours supérieur pour le système de coculture que pour la culture conventionnelle. Ceci a reflété des différences dans les préparations d'hépatocytes et est probablement une conséquence du traitement à la collagénase.

II — Cas des hépatocytes humains

Les caractéristiques morphologiques, la survivance cellulaire et les différents paramètres caractérisant les fonctions spécifiques des hépatocytes cultivés selon les deux systèmes (conventionnel et co-culture) ont été étudiés. La méthodologie générale se rapportant à l'évaluation des caractéristiques propres aux hépatocytes est identique à celle utilisée dans le cas des hépatocytes de rat, si ce n'est l'emploi d'antisérum humain pour la détection de l'albumine et de la transferrine.

Le système de co-culture d'hépatocytes humains et de cellules épithéliales de foie Chang irradiées a également permis de maintenir à la fois toutes les caractéristiques morphologiques et la survivance cellulaire pendant plus de six semaines de culture. Les hépatocytes maintiennent leur morphologie cuboïdale polyédrique typique et sont organisés en trabécules permettant la formation des canalicules biliaires. Dans le système conventionnel, les hépatocytes maintiennent leur structure typique pendant seulement 8 à 12 jours. Plus tard, les cellules dégénèrent progressivement pour disparaître presque complètement après trois semaines.

Dans le système de co-culture, les protéines spécifiquement produites par les hépatocytes, telles que l'albumine et la transferrine sont sécrétées à leur niveau optimal parallèlement au maintien des caractéristiques morphologiques. En mono-culture, les sécrétions ont par contre décliné après deux semaines pour rester stables jusqu'à la mort des hépatocytes.

L'excrétion d'urée a évolué parallèlement à la sécrétion d'albumine et à l'observation morphologique montrant également l'effet bénéfique des cellules épithéliales de foie Chang sur les hépatocytes humains en culture.

L'activité des récepteurs membranaires spécifiques des asialoglycoprotéines a également été étudiée dans les deux systèmes. Le rapport spécifique de capture par les hépatocytes, c'est-à-dire le rapport de captage de l'albumine galactosylée sur le captage de l'albumine native, est supérieur à 5 après 23 jours de co-culture alors qu'il est réduit à 1,7 après 8 jours de monoculture. 24 h après la mise en culture des hépatocytes, la valeur initiale était égale à 12.

En ce qui concerne le métabolisme médicamenteux, des hépatocytes humains en culture depuis 8 jours ont été incubés en présence d'astémizole pendant 24 h. 90 % de la drogue a été métabolisée par les hépatocytes en culture conventionnelle et la glucuronidation intervient pour seulement 25 %. Lorsque les hépatocytes sont co-cultivés par les cellules de foie Chang irradiées, plus de 90 % de l'astémizole sont métabolisés et 70 % de la drogue sont conjugués en tant que glucuronide.

Références :

(1) BISSELL, D.M. 1981. Fed. Proc., 40, 2469
(2) MICHALOPOULOS, G. & PITOT, H. 1975 Exp. Cell Res., 94, 70
(3) DESCHENES, J., VALET, J.P., MARCEAU, N. 1980. In vitro, 16,722
(4) ROJKIND, M., et al. 1980. J. Cell Biol., 87, 255
(5) GUGUEN-GUILLOUZO, C. et al. 1982, Biol. Cell, 46, 11
(6) MICHALOPOULOS, G., RUSSEL, G. & BILES, C. 1979 In vitro, 15, 796
(7) LANGENBACH, R. et al. 1979, Cancer Res., 39, 3509
(8) WANSON, J.C. et al., 1977. In : Kupffer cells and other liver sinusoïdal cells. Wisse, E. & KNOOK,

**EP 0 218 488 B1**

D.L. eds. Elsevier North Holland Biomedical Press, Amsterdam, pp. 141-150

(9) GUGUEN-GUILLOUZO, C. et al. 1983, Exp. Cell res., 143, 47
(10) GUGUEN-GUILLOUZO, C. et al. 1983
In : Isolation, Characterization, end Use of Hepatocytes
R.A. Harris & M.W. Cornell, Eds. Elsevier Science Publishing Co, Amsterdam, Netherlands, p. p. 105-110
(11) BEGUE, J.M., et al. 1983. Biochem, Pharmacol, 32, 1643
(12) BEGUE, J.M., et al. 1984. Hepatology, 4, 839
(13) GUGUEN-GUILLOUZO, C. & GUILLOUZO, A., 1983. Mol. Cellular Biochem., 5 354, 35
(14) BAFFET G. et al., 1982. Biochem. Biophys. Res. comm., 109, 507
(15) GUGUEN-GUILLOUZO, C. et al., 1984. Developmental Biology, 105, 211
(16) GRISHAM, J.W. 1980. Ann. N.Y. Acad. Sci., 349, 128
(17) ASHWELL, G. and MORELL, A.G. 1974 Adv. Enzymol., 41, 99-128

**Revendications**

1. Procédé de culture des hépatocytes, permettant de maintenir l'intégralité de leurs fonctions spécifiques, caractérisé en ce que lesdits hépatocytes sont mis en co-culture avec des cellules de foie de type épithélial dont les capacités de division ont été définitivement inhibées par un traitement approprié.

2. Procédé selon la revendication 1, caractérisé en ce que les hépatocytes sont des hépatocytes de rongeurs.

3. Procédé selon la revendication 1, caractérisé en ce que les hépatocytes sont des hépatocytes humains.

4. Procédé de culture des hépatocytes selon la revendication 1, caractérisé en ce que les cellules hépatiques de type épithélial utilisées proviennent d'une lignée dérivée de cellules de canalicules biliaires.

5. Procédé selon les revendications 1 et 4, caractérisé en ce que les cellules de foie de type épithélial sont des cellules Chang.

6. Procédé selon la revendication 5, caractérisé en ce que les cellules Chang sont cultivées en monocouche.

7. Procédé selon la revendication 1, caractérisé en ce que le traitement approprié est de nature physique.

8. Procédé selon la revendication 7, caractérisé en ce qu'il s'agit de radiations gamma.

9. Procédé selon la revendication 1, caractérisé en ce que le traitement approprié est de nature chimique.

10. Procédé selon la revendication 9, caractérisé en ce qu'il s'agit de drogues anti-mitotiques telles que la mytomycine-C.

11. Procédé selon la revendication 1, caractérisé en ce que le traitement approprié combine les radiations gamma et la mytomycine.

12. Co-cultures d'hépatocytes avec des cellules hépatiques de type épithélial, caractérisées en ce qu'elles sont obtenues par la mise en œuvre du procédé selon l'une des revendications 1 à 11.

**Claims**

1. A method for culturing hepatocytes by means of which the totality of their specific functions may be maintained, wherein the said hepatocytes are co-cultured with epithelial type liver cells whose capacity for division has been permanently inhibited by a suitable treatment.

2. The method as claimed in claim 1, wherein the hepatocytes are rodent hepatocytes.

3. The method as claimed in claim 1, wherein the hepatocytes are human hepatocytes.

4. The method for culturing hepatocytes as claimed in claim 1, wherein the epithelial type liver cells used originate from a line derived from cells of biliary canaliculi.

5. The method as claimed in claims 1 and 4, wherein the epithelial type liver cells are Chang cells.

6. The method as claimed in claim 5, wherein the Chang cells are cultured in a monolayer.

7. The method as claimed in claim 1, wherein the suitable treatment is physical in nature.

8. The method as claimed in claim 7, wherein gamma radiation is used.

9. The method as claimed in claim 1, wherein the suitable treatment is chemical in nature.

10. The method as claimed in claim 9, wherein antimitotic drugs such as mitomycin-C are used.

11. The method as claimed in claim 1, wherein the suitable treatment combines gamma radiation and mitomycin-C.

12. Co-cultures of hepatocytes with epithelial type liver cells, which cultures are obtained by carrying out the method as claimed in one of claims 1 to 11.

**Patentansprüche**

1. Verfahren zur Züchtung von Hepatozyten unter Erhaltung der Vollständigkeit ihrer spezifischen Funktionen, dadurch gekennzeichnet, daß man die Hepatozyten gemeinsam mit Leberepithelzellen, deren Fähigkeit zur Zellteilung durch eine geeignete Behandlung endgültig gehemmt wurde, züchtet.

2. Verfahren nach Anspruch 1, dadurch gekennzeichnet, daß es sich bei den Hepatozyten um Nagetierhepatozyten handelt.

3. Verfahren nach Anspruch 1, dadurch gekennzeichnet, daß es sich bei den Hepatozyten um Humanhepatozyten handelt.

4. Verfahren zur Züchtung von Hepatozyten nach Anspruch 1, dadurch gekennzeichnet, daß die verwendeten Leberepithelzellen aus einer von Gallenkapillarzellen abstammenden Linie herrühren.

5. Verfahren nach Ansprüchen 1 und 4, dadurch gekennzeichnet, daß es sich bei den Leberepithelzellen um Chang-Zellen handelt.

6. Verfahren nach Anspruch 5, dadurch gekennzeichnet, daß die Chang-Zellen einschichtig gezüchtet werden.

7. Verfahren nach Anspruch 1, dadurch gekennzeichnet, daß es sich bei der geeigneten Behandlung um eine physikalische Behandlung handelt.

8. Verfahren nach Anspruch 7, dadurch gekennzeichnet, daß man sie mit Gamma-Strahlung durchführt.

9. Verfahren nach Anspruch 1, dadurch gekennzeichnet, daß es sich bei der geeigneten Behandlung um eine chemische Behandlung handelt.

10. Verfahren nach Anspruch 9, dadurch gekennzeichnet, daß man sie mit Antimitosemitteln, wie Mytomycine-C, durchführt.

11. Verfahren nach Anspruch 1, dadurch gekennzeichnet, daß man die geeignete Behandlung als kombinierte Behandlung mit Gamma-Strahlung und Mytomycine durchführt.

12. Cokulturen von Hepatozyten mit Leberepithelzellen, dadurch gekennzeichnet, daß sie im Rahmen des Verfahrens nach einem der Ansprüche 1 bis 11 erhalten wurden.